# EUROPEAN PATENT SPECIFICATION

(11) **EP 1 352 051 B1**
(45) Date of publication and mention of the grant of the patent: **07.06.2006**
(21) Application number: 01965205.6
(22) Date of filing: 21.08.2001
(51) Int. Cl.: C12N 5/06, A01K 67/027, A61P 35/00

(54) **IN VIVO ANIMAL MODEL OF HUMAN LEUKEMIA**
IN VIVO TIERMODEL FÜR HUMANE LEUKÄMIE
MODELE ANIMAL 'IN VIVO' DE LEUCEMIE HUMAINE

(30) Priority: 22.08.2000 US 643508
(43) Date of publication of application: 15.10.2003
(73) Proprietor: Novartis AG, 4056 Basel (CH); Novartis Pharma GmbH, 1235 Vienna (AT); THE SCRIPPS RESEARCH INSTITUTE, La Jolla, CA 92037 (US)
(72) Inventor: YU, John, La Jolla, CA 92037 (US)
(74) Representative: Gros, Florent
(86) International application number: PCT/EP2001/009661
(87) International publication number: WO 2002/016559

(56) References cited:
- WO-A-01/94541
- US-A- 5 733 542
- US-A- 6 010 696
- STEELE J P C ET AL: "T-cell acute lymphoblastic leukemia (T-ALL): Engraftment in SCID mice." BLOOD, vol. 86, no. 10 SUPPL. 1, 1995, page 782A XP002200448 37th Annual Meeting of the American Society of Hematology;Seattle, Washington, USA; December 1-5, 1995 ISSN: 0006-4971
- STEELE JEREMY P C ET AL: "Growth of human T-cell lineage acute leukemia in severe combined immunodeficiency (SCID) mice and non-obese diabetic SCID mice." BLOOD, vol. 90, no. 5, 1997, pages 2015-2019, XP002200449 ISSN: 0006-4971
- DIALYNAS DENO P ET AL: "Engraftment of human T-cell acute lymphoblastic leukemia in immunodeficient NOD/SCID mice which have been preconditioned by injection of human cord blood." STEM CELLS (MIAMISBURG), vol. 19, no. 5, 2001, pages 443-452, XP002200450 ISSN: 1066-5099
- DIALYNAS DENO P ET AL: "Preconditioning with fetal cord blood facilitates engraftment of primary childhood T-cell acute lymphoblastic leukemia in immunodeficient mice." BLOOD, vol. 97, no. 10, 15 May 2001 (2001-05-15), pages 3218-3225, XP002200451 ISSN: 0006-4971
- SHAO L ET AL: "Enhancement of human leukemia colony formation by factor(s) derived from bone marrow of NOD/scid mice reconstituted with human cord blood." BLOOD, vol. 96, no. 11 Part 1, 16 November 2000 (2000-11-16), page 767a XP001073697 42nd Annual Meeting of the American Society of Hematology;San Francisco, California, USA; December 01-05, 2000 ISSN: 0006-4971
- SHAO LI-EN ET AL: "Human cord blood conditioned medium enhances leukemia colony formation in vitro." LEUKEMIA RESEARCH, vol. 24, no. 12, December 2000 (2000-12), pages 1041-1048, XP002200452 ISSN: 0145-2126

## Description

### Technical Field of the invention

The field of this invention is leukemia. More particularly, this invention pertains to models of human leukemia including an *in vivo* rodent model of human leukemia.

### Background of the Invention

T-cell acute lymphoblastic leukemia (T-ALL) comprises -20% of ALL (Kersey JH., *Blood* 1997; 90: 4243-4251), with ALL being the most common type of cancer in children. A better understanding of the biology of T-ALL at the molecular level would facilitate the development of selective therapy that exploits specific biological properties of the leukemia, thereby improving the outlook for this disease. Even though a number of leukemia cell lines of T-cell origin have been established from patients (Gjerset R, et al., *Cancer Res* 1990; 50: 10-14; Smith SD, et al., *Blood* 1978; 52: 712-718; Lange B, et al., *Blood* 1987; 70: 192-199; and Smith SD, et al., *Cancer Res* 1984; 44: 5657-5660), difficulty in maintaining primary cultures of leukemia cells from patients has impeded study of the development of the disease.

Leukemic progenitor cells have been implicated in the maintenance and expansion of leukemic blast populations (Uckun FM, et al., *Immunology* 1988; 140: 2103-2111; Uckun FM, et al., *Blood* 1990; 76: 1723-1733). These clonogenic blast cells comprise only 0.05 to 1.5% of the bulk marrow or peripheral blood blasts from ALL patients (Uckun FM, et al., *Immunology* 1988; 140: 2103-2111; Touw I, et al., *Blood* 1986; 68: 1088-1094), identified on the basis of their ability to proliferate and form colonies in semi-solid media in response to specific growth factors (Touw I, et al., *Blood* 1986; 68: 1088-1094; Touw I, et al., *Blood* 1985; 66: 556-561). It is generally assumed that the colony-forming blasts represent the *in vitro* counterparts of the *in vivo* ALL blast progenitors (Uckun FM, et al., *Immunology* 1988; 140: 2103-2111). Despite these *in vitro* studies, leukemia-initiating cells were not demonstrated *in vivo* until recently (Holyoake T, et al., *Blood* 1999; 94: 2056-2064; Ailles LE, et al., *Nat Med* 1997; 3: 730-737; and Terpstra W, et al., *Blood* 1996; 87: 2187-2194).

The ability to engraft T-ALL cells directly from patient samples into immunodeficient rodents such as Nonobese Diabetic x Severe Combined Immunodeficient (NOD/scid) mice would be uniquely valuable in this regard, as well as for predicting the clinical course of the disease, detecting residual disease, and developing individualized therapeutic strategies. The availability of a robust *in vivo* mouse model for T-ALL would expedite characterization of the corresponding leukemia-initiating cell, as well as delineation of cellular hierarchy within the leukemia. Pre-conditioning sub-lethally irradiated immunodeficient NOD/scid mice with human cord blood mononuclear cells (MNCs) facilitates the subsequent engraftment in these mice of primary T-ALL cells obtained from patients at the time of diagnosis. The present invention provides, in great detail a novel *in vivo* model of human leukemia engraftment. The data show that the level of engraftment depends on both the number of cord blood MNCs and T-ALL cells injected. In addition, the data document the fidelity of the model to the human pathology with regard to the pattern of leukemia dissemination, as well as with regard to the maintenance of the leukemia-initiating cell within the leukemia-engrafted mouse. The data also provide evidence that the cord blood pre-conditioned NOD/scid mouse is applicable to the study of other human leukemias in addition to T-ALL.

### Brief Summary of the Invention

The present invention provides a process for making an *in vivo* model of human leukemia. The process includes the steps of: pre-conditioning an immunodeficient rodent by administering to the rodent a sub-lethal dose of irradiation and injecting the rodent with an effective pre-conditioning amount of about 10⁶ to 10⁸ mononuclear cells (MNCs) derived from human fetal cord blood; maintaining the rodent for 3 to 12 days, preferably from about 5 to 10 days and, more preferably from about 6 to 9 days; and injecting the rodent with an effective engrafting amount of about 10⁶ to 10⁷ primary human leukemia cells. In one embodiment, the cord blood MNCs are stromal cells that comprise mesenchymal stem cells.

The immunodeficient rodent is preferably an immunofeficient mouse, more preferably a NOD/scid mouse. Pre-conditioning the mouse includes two steps. First, the mouse is irradiated with a sub-lethal dose of gamma radiation. The irradiation is whole body irradiation. A preferred sub-lethal radiation dose is from about 200 to about 500 rads. More preferably, the dose is from about 300 to about 400 rads and, even more preferably about 350 rads.

Immediately following irradiation, the mouse is injected with mononuclear cells from fetal cord blood from a normal human subject. The number of mononuclear cells is from about 10⁶ to about 10⁸ cells. More preferably, about 10-25 x 10⁶ cells are injected. About 1 week after pre-conditioning, the mouse is injected with viable primary human leukemia cells. The number of primary leukemia cells is from about 10⁶ to about 10⁷ cells. An especially preferred number of primary leukemia cells is 1-5 x 10⁶ cells.

In a related aspect, an *in vivo* model of human leukemia can be produced as set forth above using stromal cells derived from bone marrow. The stromal cells of the bone marrow comprise stem cells of mesenchymal nature. A process of this invention can use any stem cells, especially mesenchymal stem cells, as the pre-conditioning agent. The present invention also provides *in vivo* models of human leukemia produced by a process of this invention.

In another aspect, the present invention provides an immunodeficient rodent having engrafted human leukemia cells. That is irradiated, injected with about 10⁶ to 10⁸ mononuclear cells derived from human fetal cord blood and then injected with about 10⁶ to 10⁷ human primary leukemia cells. In another aspect of the invention the irradiated mouse is injected with about 10⁶ to 10⁸ mesenchymal stem cells derived from cord blood or bone marrow and then injected with about 10⁶ to 10⁷ human primary leukemia cells. The engrafted leukemia cells are found in the bone marrow and spleen of the mouse. Preferably, the immunodeficient rodent is an immunodeficient mouse, more preferably is an NOD/scid mouse.

The efficient engraftment and subsequent expansion of the leukemia within pre-conditioned rodents affords a viable window for addressing at the molecular level all events up to and including the expansion. The dissemination of engrafted primary leukemic cells within the pre-conditioned rodent mimics the findings for the human pathology. The level of primary cell engraftment increases both with increasing number of pre-conditioning cells (e.g., MNCs, stem cells) and with increasing number of primary leukemia cells injected.

### Brief Description of the Drawings

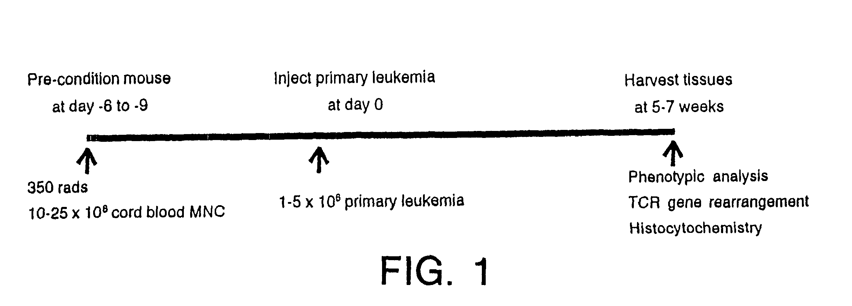

In the drawings that form a portion of the specification
Fig. 1 shows a protocol for cord blood pre-conditioning of NOD/scid mice and analysis of the engraftment of primary human leukemia.

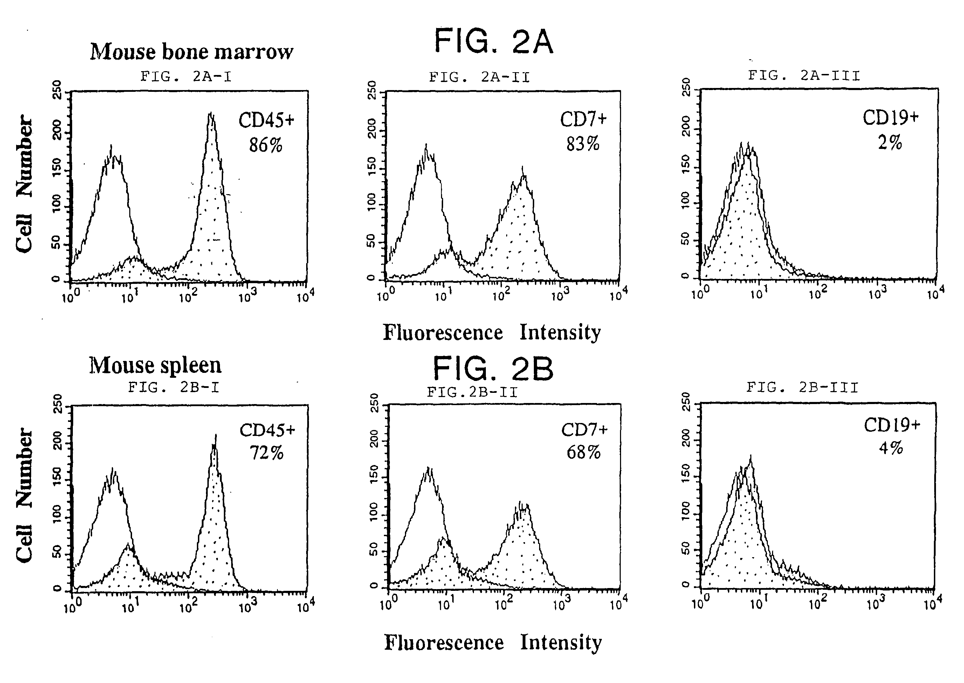

Fig. 2 shows leukemia-engrafted bone marrow and spleen from a cord blood pre-conditioned mouse injected with primary T-ALL cells. Mice were analyzed by flow cytometry for T-ALL engraftment in bone marrow (A) and spleen (B). For each panel, the filled histogram curve corresponds to the indicated experimental monoclonal antibody (mAb) and is superimposed over an open histogram corresponding to the istotype control mAb. The fraction of cells staining positive for the experimental mAb was determined by subtraction of the curves, using CeilQuest 3.2.1 software. The percentage of human CD45⁺, CD7⁺, and CD 19⁺ cells is indicated in the panels.

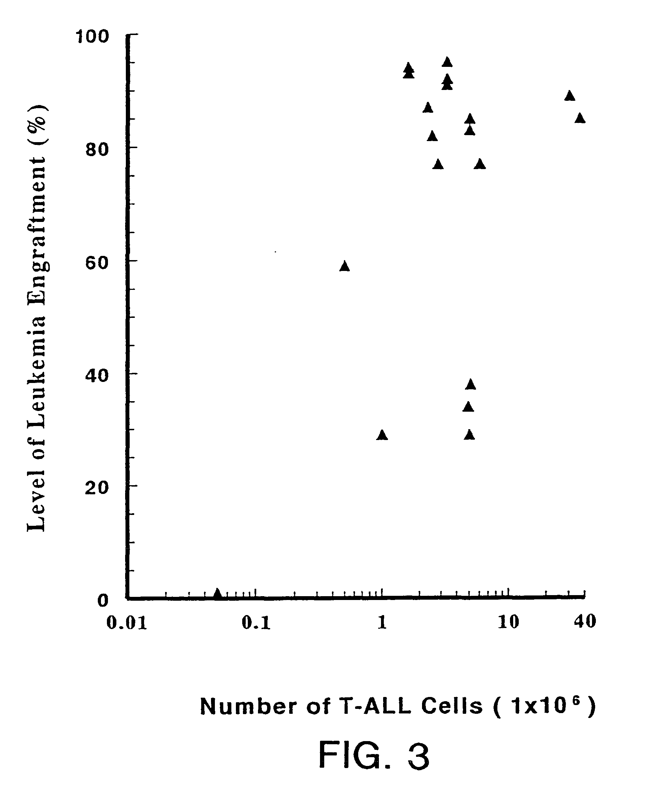

Fig. 3 shows engraftment of primary T-ALL in mouse bone marrow for a series of primary T-ALL donors. The level of T-ALL engraftment was determined by flow cytometry, on the basis of CD45, CD7, and CD5 expression.

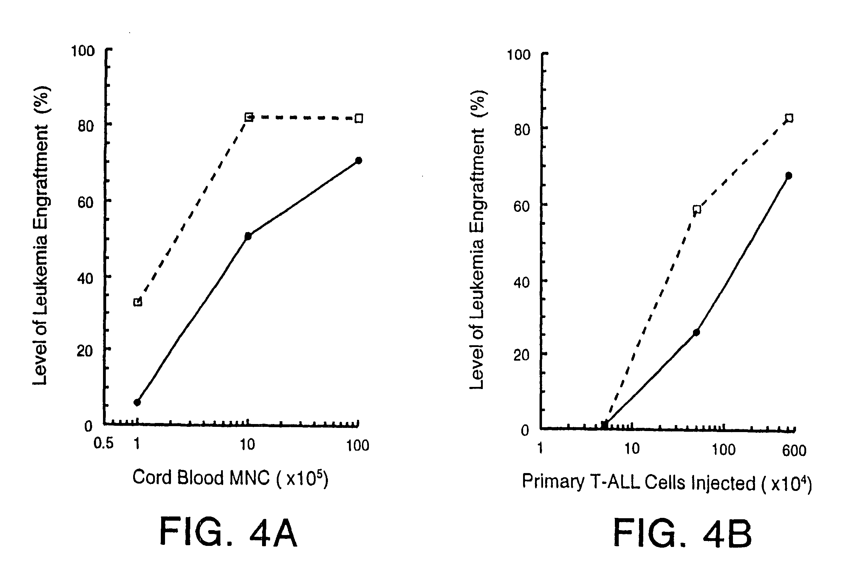

Fig. 4 shows that the level of engraftment in mouse bone marrow and spleen is dependent on both the number of cord blood MNCs and the number of T-ALL cells. In (A), mice were injected with the indicated number of cord blood MNCs 7 days prior to the injection of 2.5 x 10⁶ primary T-ALL cells; the mice were sacrificed for analysis 6 weeks after injection of the T-ALL cells. In (B), mice were injected with 10 x 10⁶ cord blood MNCs 7 days prior to the injection of the indicated number of primary T-ALL cells; the mice were sacrificed for analysis 6 weeks after injection of the T-ALL cells. The percentage of T-ALL in engrafted bone marrow (□-□-□) and spleen (•-•-•) was determined flow cytometrically as CD7⁺CD5⁺cells, the phenotype of the primary TALL in each case.

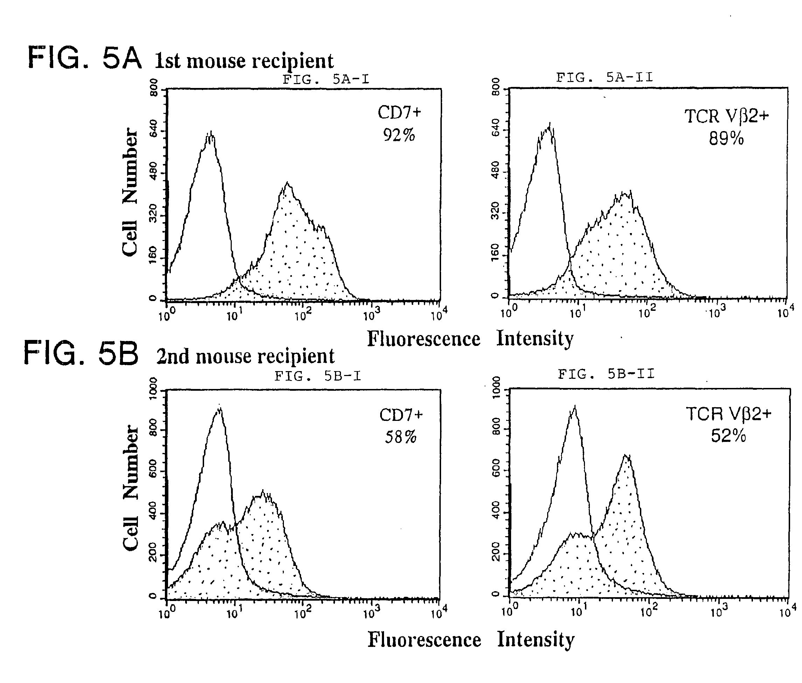

Fig. 5 shows the maintenance of the T-ALL leukemia initiating cell within the leukemia-engrafted mouse. In (A), the first mouse was injected with primary T-ALL cells obtained from a patient. In (B), however, the second mouse was injected with T-ALL cells in engrafted spleen recovered from the mouse in (A). Specifically, in (A) mice were injected with 10 x 10⁶ cord blood MNCs 9 days prior to the injection of 1.6 x 10⁶ primary T-ALL cells; the mouse was sacrificed for analysis 7 weeks after injection of the T-ALL cells. In (B) mice were injected with 25 x 10⁶ cord blood MNCs 8 days prior to the injection of the indicated number of T-ALL cells obtained from engrafted spleen recovered from the mouse in (A); the mouse was sacrificed for analysis 5 weeks after injection of the T-ALL cells. The percentage of T-ALL in engrafted bone marrow was determined flow cytometrically as CD7⁺CD5⁺ and CD7⁺Vβ2⁺ cells. The CD7 and TCR Vβ2 profiles are presented here.

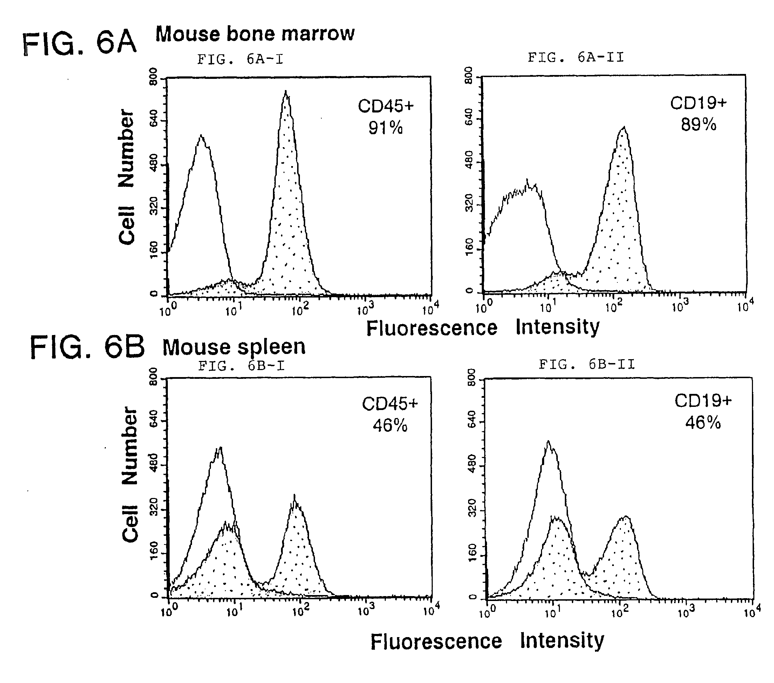

Fig. 6 shows the engraftment of primary childhood B-ALL in cord blood pre-conditioned mice. Specifically, mice were injected with 25 x 10⁶ cord blood MNCs 9 days prior to injection with 5 x 10⁶ primary B-ALL cells. The mice were sacrificed for analysis 6 weeks after injection of the B-ALL cells. The percentage of engrafted B-ALL in bone marrow (A) and spleen (B) was determined flow cytometrically as CD45⁺CD 19⁺ cells.

### Detailed Description of the Invention

The present invention provides models of leukemia including an *in vivo* animal model of human leukemia. The animal for use with the *in vivo* model is a rodent and, more particularly, a mouse. The rodent is immunodeficient. That is, the animal lacks the normal capacity to respond to an insult with an immunological response. Numerous immunodeficient rodent models are well known in the art. An especially preferred immunodeficient animal is a severe combined immunodeficient mouse (scid mouse).

Means for obtaining such scid mice are well known in the art. An especially preferred scid mouse for use in the present invention is a Nonobese Diabetic x severe combined immunodeficient (NOD/scid) mouse.

The immunodeficient animal contains engrafted human leukemia cells. As used herein, the term "engrafted" and its grammatical equivalents means transplanted cells that have migrated throughout the organism to particular tissues. Engrafted leukemia cells can be found throughout the animal model. More particularly, engrafted leukemia cells are found in the liver (portal and mesenchymal areas), kidney (perivascular and periglomerular interstitial spaces), lung (parenchymal tissue), thymus, adrenal gland and peripheral blood. The largest number of engrafted cells is found in the hemopoietic tissues, bone marrow and spleen.

An *in vivo* model of leukemia according to the present invention has a variety of uses. One, the model can be used to study leukemogenesis. Two, the model can serve as a vehicle for testing the efficacy of various treatments. Third, the model can be used as a vehicle for screening putative antileukemia therapeutic agents. Fourth, the model can serve as a mean for continuous expansion of patient's leukemia cells for diagnosis/research purposes.

A detailed description of how to make an *in vivo* animal model of human leukemia is set forth hereinafter below. NOD/scid mice (Shultz LD, et al., *J*. *Immunol* 1995; 154: 180-191) were bred and maintained in a specific pathogen-free environment at The Scripps Research Institute vivarium in sterile Micro-Isolator cages and ventilated mouse racks (Lab Products, Seaford, DE) without antibiotics. Five to six week old mice mice of either sex (but matched within a given experiment) were used in the present study.

Heparinized peripheral blood or bone marrow samples were obtained from patients with childhood T-ALL who enrolled in protocol #9400 Pediatric Oncology Group. Analogous samples were also obtained from patients with childhood B-cell acute lymphoblastic leukemia (B-ALL) or acute myeloblastic leukemia (AML). In one study, the mononuclear cell (MNC) fraction from peripheral blood/bone marrow was isolated by Ficoll-Paque density gradient separation (Pharmacia, Piscataway, NJ). The content of lymphoblasts, as determined by Wright stain, was generally >90%. In some cases, MNCs of leukemia samples were cryopreserved and stored in liquid nitrogen before use in the studies. Viability on thawing was generally greater than 80% as determined by trypan blue dye exclusion. Fetal cord blood samples were obtained from umbilical cord scheduled for discard, according to procedures approved by our Institutional Review Board. After Ficoll-Paque density gradient centrifugation, the MNCs were collected and washed with RPMI 1640 medium containing 2% fetal calf serum (FCS). Cord blood was used for injection as described below.

In a second study the umbilical cord MNCs were placed in culture, where an adherent layer of cells containing mesenchymal stem cells was observed. Human cord blood MNCs were seeded at 1.5 x 10⁶ cells/ml in 10% fetal calf serum/RPMI 1640 medium and cultured for two weeks with weekly change of medium. Certain of the cells adhered to the culture plates. These adherent cells were evaluated and found to contain mesenchymal stem cells capable of differentiating into various cells, including osteoblasts and adipocytes. These mesenchymal stem cells were isolated and used in pre-conditioning as set forth below.

The protocol for pre-conditioning of NOD/scid mice and analysis of the engraftment of primary human leukemia is outlined in Fig. 1. Prior to leukemia implantation, the mice received 350 rads total body irradiation from a ¹³⁷Cs γ- irradiator. Immediately thereafter, 10-25 x 10⁶ cells were injected in 0.25 ml sterile PBS via tail vein. Six to nine days later, 1-5 x 10⁶ viable primary leukemia cells from a patient were suspended in 0.25 ml PBS and injected via tail vein. For a given experiment, leukemia cells from a single donor were used for all mice. Experimental mice were typically set up as two to four replicates. Mice were sacrificed when they became moribund with disseminated leukemia or electively between 5 and 7 weeks after the leukemia cell injection. Necropsies were performed, and the burden of leukemia cells in mouse tissues was determined by flow cytometry and histocytochemistry as described below.

Gross examination of various mouse tissues was performed after laparatomy immediately after sacrifice. Multiple tissues from mice (including liver, kidney, lung, and brain) were fixed in aqueous buffered zinc formalin (Z-fix; Anatech, Battle Creek, MI), dehydrated, and embedded in paraffin by routine methods. Glass slides with 4 µm tissue sections were prepared and stained with hematoxylin/eosin. The bone marrow of mice was collected from femurs and tibias. A single cell suspension was prepared by gentle pipetting. Spleen cells were collected by gentle dissociation. Red blood cells within the bone marrow and spleen cell suspensions were lysed using buffered ammonium chloride. Cell debris was removed by filtration through a sterile nylon cell strainer (Becton Dickinson, San Jose, CA).

Multi-parameter analysis of single-cell suspensions from mouse bone marrow and spleen was carried out using a FACScan flow cytometer (Becton Dickinson). Two-color immunofluorescence was used to identify human leukemia cells. Fluorescein isothiocyanate (FITC)- or phycoerythrin (PE)-conjugated mouse anti-human monoclonal antibodies (mAbs) were obtained from PharMingen (San Diego, CA), with the exception of PE-conjugated anti-TCR V β2 (clone MPB2D5, Coulter, Miami, FL). The mAbs used in the work presented here include those directed against human CD5 (clone UCHT2), CD7 (M-T701), CD 19 (HIB 19), CD33 (WM53), and CD45 (HI30). During analysis, red blood cells and debris were gated out on the basis of forward angle and 90° side scatter. At least 15,000 events were collected for each sample. Istoype-matched control mAbs [FITC- or PE-conjugated IgGI (clone MOPC021)] were used to determine the appropriate cursor settings for analysis. Using CeilQuest 3.2.1 software (Becton Dickinson), data were analyzed and displayed by twodimensional plots and by one-dimensional histograms.

Pre-conditioning sub-lethally irradiated immunodeficient NOD/scid mice with human cord blood mononuclear cells (MNCs) facilitates the subsequent engraftment in these mice of primary leukemia cells obtained from patients with T-ALL. As outlined in Fig. 1, in this model irradiated NOD/scid mice are injected with human cord blood MNCs approximately 1 week prior to the injection of primary leukemia cells obtained from patients. The mice are then sacrificed approximately 6 weeks later and the level of leukemia cell engraftment determined. A typical profile of T-ALL engrafted mouse bone marrow and spleen, as assessed by flow cytometry, is presented in Fig. 2. CD45 expression is indicative of total human hematopoietic cell engraftment. CD7 is expressed by engrafted human T-ALL. CD 19 is indicative of engrafted human cells of the B cell lineage. For this experiment, CD45⁺CD7⁺ engrafted T-ALL cells comprise approximately 83% of bone marrow and 68% of spleen, as indicated by the corresponding histograms presented in Fig. 2. Notably, there are very few CD 19⁺ cells (approximately 2% in bone marrow and 4% in spleen) in the T-ALL engrafted mouse, suggesting that expansion of the T-ALL overtakes the expansion of normal CD 19⁺ cells developing from engrafted cord blood MNCs (Vormoor J, et al., *Blood* 1994; 83: 2489-2497; Hogan CJ, et al., *Blood* 1997; 90: 85-96; Kollmann TR, et al., *Immunology* 1994; 91: 8032-8036; Yu J., *I Formos Med Assoc* 1996; 95: 281-293; and Pflumio F, et al., *Blood* 1996; 88: 3731-3740). In some cases, further confirmation that the engrafted cells were derived from the injected primary T-ALL was carried out by analysis of TCR Vβ gene usage. Similar results were obtained from studies using the mesenchymal stem cells, which were shown to enhance the engraftment of human leukemia cells. Current studies also characterized the level of T-ALL engraftment in mouse bone marrow for a series of primary T-ALL donors over a range of injected T-ALL cell number (Fig. 3). In these studies, eight different primary T-ALL donors were used. Efficient engraftment in mouse bone marrow typically is observed at 6 weeks following injection of 1-5 x 10⁶ primary T-ALL cells into a mouse which has been pre-conditioned with cord blood (Fig. 3). Studies then addressed the issue of whether the level of engraftment in mouse bone marrow and spleen at 6 weeks is dependent on the number of cord blood MNCs and the number of primary T-ALL cells injected (Fig. 4). In Fig. 4, two different experiments were set up, with the same primary T-ALL donor but different cord blood donors. From Fig. 4A it is apparent that the level of T-ALL engraftment in mouse bone marrow and spleen at 6 weeks is dependent on the number of cord blood cells used for pre-conditioning. Analogously, from Fig. 4B it is apparent that the level of T-ALL engraftment in bone marrow and spleen at 6 weeks is dependent on the number of primary T-ALL cells injected.

In order to address the likely utility of the present mouse model for the study of T-ALL metastasis and the corresponding therapeutic intervention, the profile of T-ALL dissemination was determined in the engrafted, cord blood pre-conditioned mouse. In liver there were notable infiltrations of leukemia cells in portal and mesenchymal areas. In kidney, human leukemia cells are aggregated in perivascular and periglomerular interstitial spaces. In the lung, leukemia cells were detected within the parenchymal tissue. Engrafted T-ALL cells also disseminated to mouse thymus, adrenal gland, and peripheral blood. Because of its central role in leukemia formation, it was of considerable interest to determine whether in our model system the leukemia-initiating cell was maintained within the leukemia-engrafted mouse and was not, for example, exhausted in the course of T-ALL expansion *in vivo.* To address this question, it was determined whether T-ALL recovered from the engrafted spleen of a mouse injected with primary T-ALL obtained from a patient could recapitulate the leukemia on transfer to a second, cord blood pre-conditioned mouse. For this work, a primary T-ALL that expresses T cell receptor β chain variable region 2 (TCR Vβ2) was used. In this way, the engrafted T-ALL could be uniquely identified as CD7⁺V β 2⁺ cells. The results of this experiment are presented in Fig. 5 for mouse bone marrow. Injection of primary T-ALL cells obtained from the patient into pre-conditioned mice led to high-level engraftment in bone marrow, determined here at 7 weeks after the T-ALL injection (Fig. 5A). Specifically, 92% of the cells in bone marrow expressed CD7 and 89% expressed Vβ2, consistent with the existence of a CD7⁺Vβ2⁺ subset comprising approximately 90% of the bone marrow cells. In the same mouse, CD7⁺Vβ2⁺ cells (T-ALL) accounted for approximately 92% of spleen cells. The T-ALL cells in engrafted spleen were used to inject a second mouse that had been pre-conditioned with cord blood (Fig. 5B). Analysis of this second mouse recipient at 5 weeks after T-ALL injection indicated recapitulation of the leukemia, with 58% of the bone marrow cells expressing CD7 and 52% expressing TCR Vβ2 (consistent with approximately 52% of bone marrow cells having the CD7⁺Vβ2⁺ phenotype). In this second mouse recipient, the level of T-ALL engraftment in spleen (CD7⁺Vβ2⁺ cells) was 68%. These results indicate unambiguously that there is maintenance of the leukemia-initiating cell within the leukemia-engrafted mouse.

Although the model system was developed initially to facilitate study of T-ALL and the pre-clinical testing of associated therapeutic strategies, it was of interest to determine whether it could be applied to the *in vivo* study of other leukemias. To this end, cord blood preconditioned mice were injected with primary childhood B-cell acute lymphoblastoid leukemia (B-ALL) cells and the level of B-ALL engraftment in mouse bone marrow and spleen determined on day 39 after B-ALL injection (Fig. 6). Approximately 90% of mouse bone marrow cells expressed a uniform CD45⁺CD19⁺ human phenotype expected for the engrafted B-ALL. Moreover, 46% of spleen cells expressed the identical CD45⁺CD19⁺ phenotype (Fig. 6B). Preliminary work suggests that the cord blood pre-conditioned mouse may also be applicable to the *in vivo* study of acute myeloblastic leukemia (AML). In this work, 16% leukemia engraftment in bone marrow and 1% engraftment in spleen were observed for a preconditioned NOD/scid mouse injected 11 days previously with AML.

### Enhancement of leukemia colony formation by cord blood or mesenchymal stem cell conditioned medium in vitro

To characterize the nature of the enhancing activity on leukemia engraftment, colony formation of leukemia clonogenic cells was examined *in vitro* in the presence of cord blood or cord blood derived mesenchymal stem cell conditioned medium. The *in vitro* leukemia colony assay is based on the ability of leukemic clonogenic cells to proliferate and form colonies in response to growth factors such as IL-2. These leukemic "progenitor" cells have been implicated in the maintenance and expansion of leukemic blast populations.

The leukemia colony formation of primary T-ALL obtained from patients and cultured with 100 units/ml of IL-2 and 10 ng/ml of phorbol 12-myristate 13-acetate (PMA) in methylcellulose was significantly enhanced by the addition of MNC or stem cell conditioned medium, in a dose-dependent manner. The enhancement of T-ALL colony formation is as great as 4-fold by factor(s) present in the conditioned medium was substantially increased compared to that observed in the absence of conditioned medium. On microscopic analysis, there is a wide range of colony sizes in the samples to which conditioned medium was added. The colonies in the cultures were individually picked, pooled for similar sizes of colonies, and the number of leukemia cells counted. It was found that the number of cells per individual colonies in cultures with cord blood conditioned medium ranged from 250 and 285 x 10² /colony, as compared to less than 100 cells/colony observed in the absence of conditioned medium. Therefore, this increase in burst size due to the addition of cord blood conditioned medium in the cultures was in the order of several fold to more than 100-fold. To further confirm that cord blood constitutively expressed *in vitro* a factor(s), which enhances *in vitro* leukemia colony formation, a double layer agar assay was performed for leukemia samples. Some "diffusable factor(s)" secreted from irradiated cord blood in the lower agar layer significantly promoted plating efficiency of leukemia colonies in the upper layer in the absence of exogenous IL-2 and PMA. As would be expected, the number of leukemia colonies on day 14 depends both on the number of irradiated cord blood MNCs in the lower agar layer as well as on the number of input T-ALL cells in the upper agar layer. The absence of colonies when only the irradiated cord blood is cultured indicates that the colonies in the upper layer are derived from the T-ALL preparations.

These *in vitro* studies of leukemia coiony formation show that proliferation of leukemic cells was likely stimulated by the addition of MNC or stem cell conditioned medium. To confirm that the cells recovered from individual colonies in the assay were primarily of leukemic origin and not simply normal T cells from the patient's blood, we took advantage of an atypical surface phenotype of one patient's primary T-ALL. Phenotypic analysis of the primary T-All leukemia sample obtained from this patient revealed that these cells are CD7⁺ (99%), CD34⁺ (98%), CD45⁺ (2%) and negative for B cell and monocyte markers. Although these leukemic cells expressed CD7 as expected, they anomalously failed to express CD45 and uniformally expressed CD34. This aberrant phenotype thus permits unambiguous discrimination of the patient's T-ALL cells from normal T cells.

Using this primary leukemia sample, leukemia colony forming assay with the addition of cord blood conditioned medium was performed. It was shown that the addition of cord blood conditioned medium greatly enhanced leukemia colony formation of MNCs from this patient by more than three fold as expected. On day 14, leukemia colonies were individually picked. Approximately 1.4 x 10⁶ cells were recovered, and these cells were pooled for flow cytometric analysis. The vast majority of cells are uniquely CD45⁻CD7⁺), CD45⁻CD34⁺), and CD7⁺CD34⁺. Collectively, the results are consistent with approximately 90% of the harvested cells from *in vitro* cultures having the surface phenotype CD45⁻ CD7⁺CD34⁺, identical to the atypical phenotype of the primary leukemia of this patient. This analysis supports the contention that conditioned medium from cord blood stimulates the *in vitro* proliferation of primary leukemia cells from patients with T-ALL, rather than simply normal T cells within the primary T-ALL sample obtained from the patient.

Consistent with this, it was further shown by flow cytometry, that the cells from this patient in the upper layer supported by the irradiated cord blood are of leukemia origin and not simply normal T cells from the patient's blood. Specifically, approximately 80% of the harvested cells from the upper layer of the agar assay, were shown to be CD45⁻CD7⁺ phenotype, similar to the atypical phenotype of primary T-ALL from this patient.

Enhancing activity in cord blood conditioned medium appears to bind to Q Sepharose at pH 7.5 that was eluted at 500 mM NaCl, and to wheat germ agglutinin affinity column that was eluted with 200-300 mM N-acetyl-D-glucosamine, and, thus, corresponds to an acidic glycoprotein(s). Cytokine IL-15, a potent immunoregulatory cytokine, is also a T-cell growth factor that can enhance activity of antigen-specific T cells and lymphokine-activated killer cells. It was shown that 10 ng/ml of recombinant IL-15 stimulated colony formation of primary leukemia by about 80% and neutralizing anti-IL-15 antibody (up to 100 ng/ml) was sufficient to block completely the IL-15 induced enhancement of leukemia colony formation in the assay. In contrast, similar doses of neutralizing antibody (10 to 100 ng/ml) did not affect the enhancement attributable to the addition of cord blood conditioned medium in the assay. These results indicate that IL-15 cannot be the leukemia enhancing activity in the cord blood conditioned medium. Consistent with this, determination by ELISA demonstrated that our preparations of cord blood conditioned medium (a total of 22 preparations) contains only a negligible amount of IL-15 (< 0.03 ng/ml); and this amount is about 100 times less that the ED₅₀ for IL-15 biological activities (i.e. 3 ng/ml).

As the *in vitro* leukemia colony assay using methylcellulose culture includes the addition of 100 units/ml of IL-2 and 10 ng/ml of PMA, an inducer of IL-2 receptor, the enhancement of leukemia colony formation by cord blood conditioned medium was unlikely to be due simply to an increase in IL-2 or IL-2 receptor. Consistent with this, the amount of IL-2 in the cord blood conditioned medium as determined by ELISA was found to be minimal (approximately at 4.3 ± 3.9 units/ml for four different preparations), as compared to the exogenous IL-2 added in the cultures. Moreover, flow cytometric analysis using anti-IL-2 receptor mAbs (MA251 for IL-2Rα, 3D7 for IL-2Rβ, and AG184 for IL-2Rγ) indicates that cord blood conditioned medium alone has no effect on the expression of IL-2 receptor.

Mononuclear cells derived from human bone marrow were cultured as described above for cord blood MNCs. The adherent layer, containing mesenchymal stem cells, was isolated as used in both *in vitro* and *in vivo* studies as described above. Stem cells derived from bone marrow were found to be effective pre-conditioning agents in the *in vivo* model of human leukemia and to enhance leukemic colony formation *in vitro.*

As set forth above, an animal model of the present invention is suitable model of human leukemia. Thus, the model has a variety of uses. One such use is the screening of putative anti-cancer (e.g., anti-leukemic) agents. Such a putative agent is administered to the animal model and the course of leukemia followed over time. Agents can be administered according to any protocol. In addition, the agent can be administered either before or after injection of the primary leukocytes.

## Claims

1. A process for making an *in vivo* model of human leukemia comprising
a) pre-conditioning an immunodeficient rodent by administering to the rodent a sub-lethal dose of irradiation and injecting the rodent with the effective pre-conditioning amount of about 10⁶ to about 10⁸ mononuclear cells derived from human fetal cord blood;
b) maintaining the rodent from step (a) for from about 5 to 10 days;
c) injecting the rodent from step (b) with the effective engrafting amount of about 10⁶ to about 10⁷ primary human leukemia cells and
d) subsequently sacrificing the rodent.

2. The process of claim 1 wherein the immunodeficient rodent is an immunodeficient mouse.

3. The process of claim 2 wherein the immunodeficient mouse is a NOD/scid mouse.

4. The process of claim 1 wherein administering the sub-lethal dose of irradiation is accomplished by irradiating the rodent with about 350 rads of total body gamma radiation.

5. The process of claim 1 wherein the primary human leukemia cells are T-cell acute lymphoblastic leukemia (T-ALL) cells.

6. The process of claim 1 wherein the mononuclear cells are stem cells.

7. The process of claim 6 wherein the stem cells comprise mesenchymal stem cells.

8. The in *vivo* model of human leukemia produced by the process of claim 1.

9. An immunodeficient rodent having engrafted human leukemia cells that is irradiated, injected with human fetal cord blood mononuclear cells and then injected with human primary leukemia cells, obtainable by the process of claim 1.

10. The rodent of claim 9 wherein the leukemia-initiating cell is maintained within the leukemia-engrafted rodent.

11. The rodent of claim 10 that is a mouse.

12. The mouse of claim 11 that is a NOD/scid mouse.

13. The rodent of claim 9 wherein the engrafted leukemia cells are found in the bone marrow and spleen of the rodent

14. A process for making an *in vivo* model of human leukemia comprising
a) pre-conditioning an immunodeficient rodent by administering to the rodent a sub-lethal dose of irradiation and injecting the rodent with the effective pre-conditioning amount of about 10⁶ to about 10⁸ stern cells derived from bone marrow;
b) maintaining the rodent from step (a) for from about 5 to 10 days;
c) injecting the rodent from step (b) with the effective engrafting amount of about 10⁶ to about 10⁷ primary human leukemia cells and
d) subsequently sacrificing the rodent.

## Patentansprüche

1. Verfahren zur Herstellung eines in vivo Modells für eine humane Leukämie, das umfasst
a) Präkonditionlerung eines immundefizienten Nagers durch die Verabreichung einer subletalen Bestrahlungsdosis an den Nager und der Injektion der wirksamen präkonditionierenden Menge von etwa 10⁶ bis etwa 10⁸ mononukleären Zellen in den Nager, die aus Nabelschnurblut des humanen Fetus stammen,
b) Halten des Nagers aus Schritt (e) für etwa 5 bis 10 Tage,
c) Injektion des Nagers von Schritt (b) mit einer wirksamen transplantierenden Menge von etwa 10⁶ bis etwa 10⁷ primären, humanen Leukämiezellen und
d) anschließende Tötung des Nagers.

2. Verfahren nach Anspruch 1, worin der immundefiziente Nager eine immundefiziente Maus ist.

3. Verfahren nach Anspruch 2, worin die immundefiziente Maus eine NOD/scid Maus Ist.

4. Verfahren nach Anspruch 1, worin die Verabreichung einer subletalen Bestrahlungsdosis durch die Bestrahlung des Nagers mit etwa 350 rad an gesamter gamma Bestrahlung des Körpers erreicht wird.

5. Verfahren nach Anspruch 1, worin die primären humanen Leukämiezellen T-Zellen einer akuten lymphoblastischen Leukämie sind (T-ALL).

6. Verfahren nach Anspruch 1, worin die mononuklären Zellen Stammzellen sind.

7. Verfahren nach Anspruch 6, worin die Stammzellen mesenchymale Stammzellen umfassen.

8. In vivo Modell der humanen Leukämie, das durch das Verfahren von Anspruch 1 hergestellt wurde.

9. Immundeflzlenter Nager mit transplantierten humanen Leukämiezellen, der bestrahlt ist, mit mononukleären Zellen, die aus Nabelschnurblut des humanen Fetus stammen, injiziert wurde und dann mit humanen, primären Leukämiezellen injiziert wurde, und durch das Verfahren von Anspruch 1 erhältlich ist.

10. Nager nach Anspruch 9. worin die Leukämie-initilerende Zelle im Leukämie-transplantlerten Nager gehalten wird.

11. Nager nach Anspruch 10, der eine Maus ist.

12. Maus nach Anspruch 11, dle eine NOD/scid Maus ist.

13. Nager nach Anspruch 9, worin die transplantierten Leukämiezellen im Knochenmark und der Milz des Nagers gefunden werden.

14. Verfahren zur Herstellung eines in vivo Modells für eine humane Leukämie, das umfasst
a) Präkonditionierung eines immundefizienten Nagers durch die Verabreichung einer subletalen Bestrahlungsdosis an den Nager und der Injektion der wirksamen präkonditionierenden Menge von etwa 10⁶ bis etwa 10⁸ Stammzellen in den Nager, die aus Knochenmark stammen,
b) Halten des Nagers aus Schritt (a) für etwa 5 bis 10 Tage,
c) Injektion des Nagers von Schritt (b) mit einer wirksamen transplantierenden Menge von etwa 10⁶ bis etwa 10⁷ primären, humanen Leukämiezellen und
d) anschließende Tötung des Nagers.

## Revendications

1. Procédé de préparation d'un modèle de leucémie humaine *in vivo,* comprenant :
a) le pré-conditionnement d'un rongeur immunodéficient en administrant au rongeur une dose d'irradiation sous-létale, et en injectant au rongeur la quantité de pré-conditionnement efficace d'environ 10⁶ à environ 10⁸ cellules mononucléées dérivées de sang de cordon foetal humain ;
b) le maintien du rongeur de l'étape (a) pendant environ 5 à 10 jours ;
c) l'injection au rongeur de l'étape (b) d'une quantité efficace pour la prise de greffe d'environ 10⁶ environ 10⁷ cellules primaires de leucémie humaine, et
d) le sacrifie ultérieur du rongeur.

2. Procédé selon la revendication 1, dans lequel le rongeur immunodéficient est une souris immunodéficiente.

3. Procédé selon la revendication 2, dans lequel la souris immunodéficiente est une souris NOD/scid.

4. Procédé selon la revendication 1, dans lequel l'administration de la dose d'irradiation sous-létale est réalisée par irradiation du rongeur avec environ 350 rad de rayonnement gamma corporel total.

5. Procédé selon la revendication 1, dans lequel les cellules primaires de leucémie humaine sont des cellules de leucémie lymphoblastique aiguë à cellules T (T-ALL).

6. Procédé selon la revendication 1, dans lequel les cellules mononucléées sont des cellules souches.

7. Procédé selon la revendication 6, dans lequel les cellules souches comprennent des cellules souches mésenchymateuses.

8. Modèle *in vivo* de leucémie humaine préparé par le procédé selon la revendication 1.

9. Rongeur immunodéficient portant des cellules de leucémie humaine greffées, qui est irradié, auquel on injecte des cellules mononucléées de sang de cordon foetal humain et auquel on injecte des cellules primaires de leucémie humaine, que l'on peut obtenir par le procédé selon la revendication 1.

10. Rongeur selon la revendication 9, dans lequel la cellule initiant la leucémie est maintenue à l'intérieur du rongeur ayant subi une greffe de leucémie.

11. Rongeur selon la revendication 10, qui est une souris.

12. Souris selon la revendication 11, qui est une souris NOD/scid.

13. Rongeur selon la revendication 9, dans lequel les cellules de leucémie greffées se trouvent dans la moelle osseuse et la rate du rongeur.

14. Procédé de préparation d'un modèle de leucémie humaine *in vivo*, comprenant :
a) le pré-conditionnement d'un rongeur immunodéficient en administrant au rongeur une dose d'irradiation sous-létale, et en injectant au rongeur une quantité de pré-conditionnement efficace d'environ 10⁶ à environ 10⁸ cellules souches dérivés de moelle osseuse ;
b) le maintien du rongeur de l'étape (a) pendant environ 5 à 10 jours ;
c) l'injection au rongeur de l'étape (b) d'une quantité efficace pour la prise de greffe d'environ 10⁶ environ 10⁷ cellules primaires de leucémie humaine, et
d) le sacrifie ultérieur du rongeur.
